# EUROPEAN PATENT APPLICATION

(11) **EP 3 907 284 A1**
(43) Date of publication of application: **10.11.2021**
(21) Application number: 20173282.3
(22) Date of filing: 06.05.2020
(51) Int. Cl.: C12N 9/10, A61K 39/00, A61P 35/02, C07K 14/725, C07K 16/30

(54) **PEPTIDES AND COMBINATIONS OF PEPTIDES FOR USE IN IMMUNOTHERAPY AGAINST HEMATOLOGIC NEOPLASMS AND OTHER CANCERS**

(71) Applicant: Eberhard Karls Universität Tübingen Medizinische Fakultät, 72074 Tübingen (DE)
(72) Inventor: Walz, Juliane, 72074 Tuebingen (DE); Nelde, Annika, 72070 Tuebingen (DE); Rammensee, Hans-Georg, 72070 Unterjesingen (DE)
(74) Representative: Witte, Weller & Partner Patentanwälte mbB

(57) **Abstract**

The present invention relates to peptides, proteins, nucleic acids and cells for use in immunotherapeutic methods. In particular, the present invention relates to the immunotherapy of cancer. The present invention furthermore relates to tumor-associated T cell peptide epitopes, alone or in combination with other tumor-associated peptides that can for example serve as active pharmaceutical ingredients of vaccine compositions that stimulate anti-tumor immune responses, or to stimulate T cells *ex vivo* and transfer into patients. Peptides bound to molecules of the major histocompatibility complex (MHC), or peptides as such, can also be targets of antibodies, soluble T cell receptors, and other binding molecules.

## Description

The present invention relates to peptides, proteins, nucleic acids and cells for use in immunotherapeutic methods. In particular, the present invention relates to the immunotherapy of cancer. The present invention furthermore relates to tumor-associated T-cell peptide epitopes that can for example serve as active pharmaceutical ingredients of vaccine compositions that stimulate anti-tumor immune responses, or to stimulate T cells *ex vivo* and transfer into patients. Peptides bound to molecules of the major histocompatibility complex (MHC), or peptides as such, can also be targets of antibodies, soluble T-cell receptors, and other binding molecules.

The present invention relates to several novel peptide sequences and their variants that can be used in vaccine compositions for eliciting anti-tumor immune responses, or as targets for the development of pharmaceutically/immunologically active compounds and cells.

### FIELD OF THE INVENTION

The present invention relates to the field of molecular biology, more particular to the field of molecular immunology.

### BACKGROUND OF THE INVENTION

Cancer is the second leading cause of death globally, accounting for an estimated 9.6 million deaths, or one in six deaths, in 2018. One in 5 men and one in 6 women worldwide develop cancer during their lifetime, and one in 8 men and one in 11 women die from the disease. Worldwide, the total number of people who are alive within 5 years of a cancer diagnosis, called the 5-year prevalence, is estimated to be 43.8 million. Lung, prostate, colorectal, stomach and liver cancer are the most common types of cancer in men, while breast, colorectal, lung, cervical and thyroid cancer are the most common among women.

The increasing cancer burden is due to several factors, including population growth and ageing as well as the changing prevalence of certain causes of cancer linked to social and economic development. This is particularly true in rapidly growing economies, where a shift is observed from cancers related to poverty and infections to cancers associated with lifestyles more typical of industrialized countries.

Within the group of leukemia, the current invention specifically focuses on chronic lymphocytic leukemia (CLL) and other hematologic neoplasms.

Chronic lymphocytic leukemia (CLL) is the most common leukemia in adults in western countries and is defined as a clonal expansion of small, monomorphic B cells, which infiltrate the bone marrow, peripheral blood and often the lymph nodes. CLL is mainly a disease occurring in elderly people, with a median age at diagnosis of 70 years. Therefore, beside clinical, molecular and cytogenetic disease characteristics, patient related factors such as age and burden of comorbidity play a role for the patient's prognosis and also for treatment decisions.

For many years, standard first-line CLL treatment was based on immunochemotherapy, e.g. the administration of anti-CD20 antibodies in combination with cytostatic agents such as fludarabine and cyclophosphamide, chlorambucil or bendamustin. However, even after initially high response rates almost all CLL patients experience disease relapse after classical chemoimmunotherapy. This is mostly due to the persistence of residual treatment resistant CLL cells after therapy (minimal residual disease, MRD), which ultimately cause disease relapse.

Therefore, based on an increasing understanding of the disease biology, in recent years, several novel approaches using small molecules directly targeting the CLL cells (e.g. ibrutinib, idealisib and venetoclax) have been established and approved for CLL therapy. These targeted therapies show high response and remission rates. The impact on disease control by ibrutinib has even led to approval of ibrutinib treatment as first line therapy. Ibrutinib is an inhibitor of the Brutons tyrosine kinase for oral application and therefore represents a well-tolerated treatment option especially for older CLL patients. Even in the relapsed disease stage, ibrutinib treatment could achieve response rates up to 63% in CLL patients. However, in most cases only partial remissions are achieved and so far, unlimited, permanent ibrutinib treatment bearing the risk of side effects and development of drug resistance is required.

Therefore, current efforts are now focusing on the further elimination of MRD to allow for a reduced treatment time and as well as the achievement of long-lasting remission and potential cure in the future. Several trials have been proposed aiming to improve MRD negativity by administration of already approved agents for CLL using various novel combinations and application sequences of chemotherapy, antibody-based immunotherapy and small molecules (e.g. NCT02802943). All these studies reported promising results with high response and remission rates but on the other hand not negligible side effects, especially cytopenia and infections, representing especially in an elderly and comorbid patient collective a significant limitation. Therefore, novel low side effect combinatorial therapies are required to improve response and remission rates without markedly increasing treatment toxicity, especially for the cohort of elderly and comorbid CLL patients.

One option to achieve this goal may be T cell-based immunotherapy, which harnesses the immune system to eliminate malignant cells. The immunogenicity of CLL, which is documented by graft-versus-leukemia effects after hematopoietic stem cell transplantation and by cases of spontaneous remissions after viral infections, as well as the favorable immune effector-to-target cell ratios in the MRD setting, suggest that CLL might be effectively targeted by T cell-based immunotherapy.

In recent years the breakthrough clinical success of T cell based immunotherapy approaches including immune checkpoint inhibitors, CAR T cells, bispecific antibodies and adoptive T cell transfer have revolutionized treatment of solid tumors and hematological malignancies (HM). However, some patients do not respond to available therapeutic strategies at all, others for limited time only. Remaining challenges to augment efficacy of T cell-based immunotherapy are particularly to improve specificity and increase the frequency of anti-tumor immune responses, to reduce toxicity and to expand the spectrum of targetable cancer entities.

A rational and promising approach to achieve this goal is peptide-based immunotherapy, which represents a low side-effect approach relying on specific immune recognition of tumor-associated HLA (human leucocyte antigen) presented peptides. Several peptide vaccination studies in solid tumors (Weihrauch et al., Phase I/II combined chemoimmunotherapy with carcinoembryonic antigen-derived HLA-A2-restricted CAP-1 peptide and irinotecan, 5-fluorouracil, and leucovorin in patients with primary metastatic colorectal cancer, Clin. Cancer Res. 11(16):5993-6001 (2005); Peoples et al., Clinical trial results of a HER2/neu (E75) vaccine to prevent recurrence in high-risk breast cancer patients, J. Clin. Oncol. 23(30):7536-7545 (2005); Walter et al., Multipeptide immune response to cancer vaccine IMA901 after single-dose cyclophosphamide associates with longer patient survival, Nat. Med. (2012); Feyerabend et al., Novel multi-peptide vaccination in Hla-A2+ hormone sensitive patients with biochemical relapse of prostate cancer, Prostate 69(9):917-927 (2009) and in HM (Mailander et al., Complete remission in a patient with recurrent acute myeloid leukemia induced by vaccination with WT1 peptide in the absence of hematological or renal toxicity. Leukemia 18(1):165-166 (2004); Oka et al., Induction of WT1 (Wilms' tumor gene)-specific cytotoxic T lymphocytes by WT1 peptide vaccine and the resultant cancer regression, Proc. Natl. Acad. Sci. USA. 101 (38):13885-13890 (2004); Van Tendeloo et al., Induction of complete and molecular remissions in acute myeloid leukemia by Wilms' tumor 1 antigen-targeted dendritic cell vaccination, Proc. Natl. Acad. Sci. USA. 107(31):13824-13829 (2010); Schmitt et al., RHAMM-R3 peptide vaccination in patients with acute myeloid leukemia, myelodysplastic syndrome, and multiple myeloma elicits immunologic and clinical responses. Blood 111(3):1357-1365 (2008)) have reported promising result in terms of *in vivo* immunogenicity of the applied peptides and also revealed clinical responses that however were limited to single patients.

Kowalewski et al., HLA ligandome analysis identifies the underlying specificities of spontaneous antileukemia immune responses in chronic lymphocytic leukemia (CLL), Proc. Natl. Acad. Sci. USA. 112(2):E166-175 (2015), describe CLL-associated antigens which are represented in HLA ligandomes of patients undergoing standard chemo-/immunotherapy. The authors refer to these peptides as pathophysiologically relevant tumor antigens and propose their implementation in cancer immunotherapy.

Further immunotherapeutic peptide-based approaches against CLL are disclosed in WO 2018/189152 and WO 2018/189148.

None of the approaches ever reached broad clinical application and efficacy, let alone approval. This may have its reason in the relative unsuitability of the described peptides to elicit CLL-specific immune responses in the patients.

It is, therefore, an object underlying the invention to provide tumor-associated T-cell peptide epitopes, which can be used to develop improved medicaments and methods for the diagnosis, prophylaxis and treatment of cancer, in particular of hematologic neoplasms such as CLL.

The present invention satisfies theses and other needs.

### SUMMARY OF THE INVENTION

The present invention provides a peptide comprising an amino acid sequence selected from the group consisting of SEQ ID NO: 1 to SEQ ID NO: 20, and variant sequences thereof which are at least 88% homologous to SEQ ID NO: 1 to SEQ ID NO: 20, and wherein said variant binds to molecule(s) of the major histocompatibility complex (MHC) and/or induces T cells cross-reacting with said variant peptide; and a pharmaceutical acceptable salt thereof, wherein said peptide is not a full-length polypeptide.

The inventors have realized that the strategy used in the art for identifying tumor antigens on the basis of gene expression analyzes and *in silico* prediction of the resulting HLA ligands has significant drawbacks. This approach is decisively hindered by the distorted correlation between gene expression and HLA-restricted antigen presentation. I.e. it cannot be predicted from gene expression whether or not an antigen is actually presented on the cell surface of tumor cells.

The inventors, therefore, applied a novel strategy. The inventors have based the selection of widely applicable peptides on the frequency of their presentation within an examined patient cohort. The inventors were, therefore, able to identify the peptides according to the invention by direct analysis of the HLA ligands of primary tumor patient samples, such as CLL samples.

The peptides according to the invention were selected on the basis of a direct comparative immunopeptidoma analysis of 52 primary CLL patients and a large HLA ligand domain database that includes more than 350 immunopeptidomas of various benign tissues, including B cells, bone marrow, CD34⁺ progenitor cells and a wide range of solid organs (Marcu et al., The HLA Ligand Atlas. A resource of natural HLA ligands presented on benign tissues, bioRxiv 778944; doi: https://doiorg/101101/778944. (2019)).

Based on data from an ongoing study, the HLA allotypes A*02, A*24 and B*07 were selected by the inventors as the most common HLA allotypes in the CLL cohort. All selected peptides were identified with high frequency and exclusively in the cohort immunopeptidome, whereas they were never identified in the "healthy" immunopeptidome database.

In addition, the potential of the peptides according to the invention for inducing functional T cell responses was determined for all peptides either by characterizing spontaneous T cell responses in cancer patients, especially suffering from CLL, using IFNy ELISPOT and intracellular cytokine staining or by *in vitro* priming with healthy T cells from donors and cancer or CLL patients, respectively.

The following table shows the peptides according to the present invention, their respective SEQ ID NOs, the HLA restriction, and the prospective source genes/proteins or full-length polypeptide from which the peptides are derived.

**Table 1: Peptides according to the invention**

| **Sequence** | **HLA restriction** | **Peptide length** | **Uniprot** | **Protein** | **Protein name** | **SEQ ID No.** |
|---|---|---|---|---|---|---|
| QPPDWLQGHYLVVRYEDL | class II | 18 | Q9Y4C5 | CHST2 | Carbohydrate sulfotransferase 2 | 1 |
| YPDRPGWLRYIQRTPYSDG | class II | 19 | 043556 | SGCE | Epsilon-sarcoglycan | 2 |
| AKPEASFQVWNKDSSSKNLIPR | class II | 22 | P15907 | SIAT1 | Beta-galactoside alpha-2,6-sialyltransferase 1 | 3 |
| DHAQLVAIKTLKDYNNPQ | class II | 18 | Q01973 | ROR1 | Tyrosine-protein kinase transmembrane receptor ROR1 | 4 |
| EPNKKFFELVGRTFDWH | class II | 17 | 014792 | HS3S1 | Heparan sulfate glucosamine 3-O-sulfotransferase 1 | 5 |
| LLLILRDPSERVLSDY | class II | 16 | 014792 | HS3S1 | Heparan sulfate glucosamine 3-O-sulfotransferase 1 | 6 |
| GYMPYLNRF | A*24 | 9 | Q9UH65 | SWP70 | Switch-associated protein 70 | 7 |
| KYSKALIDYF | A*24 | 10 | P51826 | AFF3 | AF4/FMR2 family member 3 | 8 |
| VYHSDIPKW | A*24 | 9 | P15907 | SIAT1 | Beta-galactoside alpha-2,6-sialyltransferase 1 | 9 |
| FYTLIPHDF | A*24 | 9 | P09874 | PARP1 | Poly [ADP-ribose] polymerase 1 | 10 |
| IYGKDVFEAF | A*24 | 10 | Q13620 | CUL4B | Cullin-4B | 11 |
| IFLTKSTKL | A*24 | 9 | P01871;P04220 | IGHM | Immunoglobulin heavy constant mu | 12 |
| RHTGALPLF | A*24 | 9 | 060292 | SI1L3 | Signal-induced proliferation-associated 1-like protein 3 | 13 |
| RPKENVTIM | B*07 | 9 | Q9HBG7 | LY9 | T-lymphocyte surface antigen Ly-9 | 14 |
| LPRLEALDL | B*07 | 9 | Q9NR96 | TLR9 | Toll-like receptor 9 (CD289) | 15 |
| SPGGAHSNL | B*07 | 9 | I3L1I5 | ARHGI | Rho guanine nucleotide exchange factor 18 | 16 |
| VPRNLPSSL | B*07 | 9 | Q9NR96 | TLR9 | Toll-like receptor 9 (CD289) | 17 |
| LIWPLLSTV | A*02 | 9 | Q99567 | NUP88 | Nuclear pore complex protein Nup88 | 18 |
| VIAELPPKV | A*02 | 9 | P01871;P04220 | IGHM | Immunoglobulin heavy constant mu | 19 |
| ALHRPDVYL | A*02 | 9 | P01871;P04220 | IGHM | Immunoglobulin heavy constant mu | 20 |

In the event of discrepancies between the sequences specified in Table 1 and those specified in the sequence listing, the information in the sequence listing takes precedence and applies.

The term "peptide" is used herein to designate a series of amino acid residues, connected one to the other typically by peptide bonds between the alpha-amino and carbonyl groups of the adjacent amino acids. The peptides are preferably between 7 and 12 amino acids in length, further preferably between 8 and 11, but can be as long as 5, 6, 7, 8, 9, 10, 11, 12, 13, 14, 15, 16, 17, 18, 19, 20, 21 or longer.

Furthermore, the term "peptide" shall include salts of a series of amino acid residues, connected one to the other typically by peptide bonds between the alpha-amino and carbonyl groups of the adjacent amino acids. Preferably, the salts are pharmaceutical acceptable salts of the peptides, such as, for example, the chloride or acetate (trifluoroacetate) salts. It has to be noted that the salts of the peptides according to the present invention differ substantially from the peptides in their state(s) *in vivo,* as the peptides are not salts *in vivo.*

The term "peptide" shall also include "oligopeptide". The term "oligopeptide" is used herein to designate a series of amino acid residues, connected one to the other typically by peptide bonds between the alpha-amino and carbonyl groups of the adjacent amino acids. The length of the oligopeptide is not critical to the invention, as long as the correct epitope or epitopes are maintained therein. The oligopeptides are typically less than about 30 amino acid residues in length, and greater than about 15 amino acids in length.

The term "peptide" shall also include "polypeptide". The term "polypeptide" designates a series of amino acid residues, connected one to the other typically by peptide bonds between the alpha-amino and carbonyl groups of the adjacent amino acids. The length of the polypeptide is not critical to the invention as long as the correct epitopes are maintained. In contrast to the terms peptide or oligopeptide, the term polypeptide is meant to refer to molecules containing more than about 30 amino acid residues.

By a "variant" of the given amino acid sequence the inventors mean that the side chains of, for example, one or two of the amino acid residues are altered (for example by replacing them with the side chain of another naturally occurring amino acid residue or some other side chain) such that the peptide is still able to bind to an MHC molecule in substantially the same way as a peptide consisting of the given amino acid sequence in consisting of SEQ ID NO: 1 to SEQ ID NO: 20. For example, a peptide may be modified so that it at least maintains, if not improves, the ability to interact with and bind to the binding groove of a suitable MHC molecule, such as HLA-A*02, and in that way, it at least maintains, if not improves, the ability to bind to the TCR of activated T cells.

The original (unmodified) peptides as disclosed herein can be modified by the substitution of one or more residues at different, possibly selective, sites within the peptide chain, if not otherwise stated. Preferably those substitutions are located at the end of the amino acid chain. Such substitutions may be of a conservative nature, for example, where one amino acid is replaced by an amino acid of similar structure and characteristics, such as where a hydrophobic amino acid is replaced by another hydrophobic amino acid. Even more conservative would be replacement of amino acids of the same or similar size and chemical nature, such as where leucine is replaced by isoleucine. In studies of sequence variations in families of naturally occurring homologous proteins, certain amino acid substitutions are more often tolerated than others, and these are often show correlation with similarities in size, charge, polarity, and hydrophobicity between the original amino acid and its replacement, and such is the basis for defining "conservative substitutions". Conservative substitutions are herein defined as exchanges within one of the following five groups: Group 1-small aliphatic, nonpolar or slightly polar residues (Ala, Ser, Thr, Pro, GIY); Group 2-polar, negatively charged residues and their amides (Asp, Asn, Glu, Gln); Group 3-polar, positively charged residues (His, Arg, Lys); Group 4-large, aliphatic, nonpolar residues (Met, Leu, IIe, Val, Cys); and Group 5-large, aromatic residues (Phe, Tyr, Trp). Less conservative substitutions might involve the replacement of one amino acid by another that has similar characteristics but is somewhat different in size, such as replacement of an alanine by an isoleucine residue. Highly non-conservative replacements might involve substituting an acidic amino acid for one that is polar, or even for one that is basic in character. Such "radical" substitutions cannot, however, be dismissed as potentially ineffective since chemical effects are not totally predictable and radical substitutions might well give rise to serendipitous effects not otherwise predictable from simple chemical principles. Of course, such substitutions may involve structures other than the common L-amino acids. Thus, D-amino acids might be substituted for the L-amino acids commonly found in the antigenic peptides of the invention and yet still be encompassed by the disclosure herein. In addition, non-standard amino acids (i.e., other than the common naturally occurring proteinogenic amino acids) may also be used for substitution purposes to produce immunogens and immunogenic polypeptides according to the present invention.

If substitutions at more than one position are found to result in a peptide with substantially equivalent or greater antigenic activity as defined below, then combinations of those substitutions will be tested to determine if the combined substitutions result in additive or synergistic effects on the antigenicity of the peptide. At most, no more than 4 positions within the peptide would be simultaneously substituted.

The amino acid residues that do not substantially contribute to interactions with the T cell receptor can be modified by replacement with other amino acids whose incorporation do not substantially affect T cell reactivity and does not eliminate binding to the relevant MHC.

Longer (elongated) peptides may also be suitable. It is possible that MHC class I epitopes, although usually between 8 and 11 amino acids long, are generated by peptide processing from longer peptides or proteins that include the actual epitope. It is preferred that the residues that flank the actual epitope are residues that do not substantially affect proteolytic cleavage necessary to expose the actual epitope during processing.

The peptides of the invention can be elongated by up to four amino acids, that is 1, 2, 3 or 4 amino acids can be added to either end in any combination between 4:0 and 0:4. Combinations of the elongations according to the invention can be found in Table 2.

**Table 2: Combinations of the elongations of peptides of the invention**

| C-terminus | N-terminus |
|---|---|
| 4 | 0 |
| 3 | 0 or 1 |
| 2 | 0 or 1 or 2 |
| 1 | 0 or 1 or 2 or 3 |
| 0 | 0 or 1 or 2 or 3 or 4 |

| N-terminus | C-terminus |
|---|---|
| 4 | 0 |
| 3 | 0 or 1 |
| 2 | 0 or 1 or 2 |
| 1 | 0 or 1 or 2 or 3 |
| 0 | 0 or 1 or 2 or 3 or 4 |

The amino acids for the elongation/extension can be from the peptides of the original sequence of the protein or any other amino acid(s). The elongation can be used to enhance the stability or solubility of the peptides.

Thus, the epitopes of the present invention may be identical to naturally occurring tumor-associated or tumor-specific epitopes or may include epitopes that differ by no more than four residues from the reference peptide, as long as they have substantially identical antigenic activity.

In an alternative embodiment, the peptide is elongated on either or both sides by more than 4 amino acids, preferably to a total length of up to 30 amino acids. This may lead to MHC class II binding peptides. Binding to MHC class II can be tested by methods known in the art.

Accordingly, the present invention provides peptides and variants of MHC class I epitopes, wherein the peptide or variant has an overall length of between 8 and 100, preferably between 8 and 30, and most preferred between 8 and 14, namely 8, 9, 10, 11, 12, 13, 14 amino acids, in case of the elongated class II binding peptides the length can also be 15, 16, 17, 18, 19, 20, 21 or 22 or 23 amino acids.

Of course, the peptide or variant according to the present invention will have the ability to bind to a molecule of the human major histocompatibility complex (MHC) class I or II. Binding of a peptide or a variant to a MHC complex may be tested by methods known in the art.

Preferably, when the T cells specific for a peptide according to the present invention are tested against the substituted peptides, the peptide concentration at which the substituted peptides achieve half the maximal increase in lysis relative to background is no more than about 1 mM, preferably no more than about 1 µM, more preferably no more than about 1 nM, and still more preferably no more than about 100 pM, and most preferably no more than about 10 pM. It is also preferred that the substituted peptide be recognized by T cells from more than one individual, at least two, and more preferably three individuals.

A person skilled in the art will be able to assess, whether T cells induced by a variant of a specific peptide will be able to cross-react with the peptide itself (Appay et al., 2006; Colombetti et al., Eur. J. Immunol. 36: 1805-1814 (2006); Fong et al., Proc. Natl. Acad. Sci. U.S.A. 98: 8809-8814 (2001); Zaremba et al., Cancer Res. 57: 4570-4577 (1997)).

These T cells can subsequently cross-react with cells and kill cells that express a polypeptide that contains the natural amino acid sequence of the cognate peptide as defined in the aspects of the invention. As can be derived from the scientific literature and databases (Rammensee et al., Immunogenetics 50: 213-219 (1999); Godkin et al., Int. Immunol 9: 905-911 (1997)), certain positions of HLA binding peptides are typically anchor residues forming a core sequence fitting to the binding motif of the HLA receptor, which is defined by polar, electrophysical, hydrophobic and spatial properties of the polypeptide chains constituting the binding groove. Thus, one skilled in the art would be able to modify the amino acid sequences set forth in SEQ ID NO: 1 to SEQ ID NO: 20, by maintaining the known anchor residues, and would be able to determine whether such variants maintain the ability to bind MHC class I or II molecules. The variants of the present invention retain the ability to bind to the TCR of activated T cells, which can subsequently cross-react with and kill cells that express a polypeptide containing the natural amino acid sequence of the cognate peptide as defined in the aspects of the invention.

In the present invention, the term "homologous" refers to the degree of identity between sequences of two amino acid sequences, i.e. peptide or polypeptide sequences. The aforementioned "homology" is determined by comparing two sequences aligned under optimal conditions over the sequences to be compared. Such a sequence homology can be calculated by creating an alignment using, for example, the ClustalW algorithm. Commonly available sequence analysis software, more specifically, Vector NTI, GENETYX or other tools are provided by public databases.

"Percent identity" or "percent identical" in turn, when referring to a sequence, means that a sequence is compared to a claimed or described sequence after alignment of the sequence to be compared (the "Compared Sequence") with the described or claimed sequence (the "Reference Sequence"). The percent identity is then determined according to the following formula: percent identity = 100 [1 -(C/R)]
wherein C is the number of differences between the Reference Sequence and the Compared Sequence over the length of alignment between the Reference Sequence and the Compared Sequence, wherein
(i) each base or amino acid in the Reference Sequence that does not have a corresponding aligned base or amino acid in the Compared Sequence and
(ii) each gap in the Reference Sequence and
(iii) each aligned base or amino acid in the Reference Sequence that is different from an aligned base or amino acid in the Compared Sequence, constitutes a difference and
(iiii) the alignment has to start at position 1 of the aligned sequences;
and R is the number of bases or amino acids in the Reference Sequence over the length of the alignment with the Compared Sequence with any gap created in the Reference Sequence also being counted as a base or amino acid.

If an alignment exists between the Compared Sequence and the Reference Sequence for which the percent identity as calculated above is about equal to or greater than a specified minimum Percent Identity then the Compared Sequence has the specified minimum percent identity to the Reference Sequence even though alignments may exist in which the herein above calculated percent identity is less than the specified percent identity.

According to the invention "full-length polypeptide" refers to the source proteins from which the peptides are derived. Full-length polypeptides are also referred to as the source genes/proteins from which the peptides are derived. The source proteins or full-length polypeptides may or may not be highly over-expressed in cancer compared with normal tissues. "Normal tissues" in relation to this invention shall mean either healthy peripheral blood mononuclear cells (PBMC) cells or other normal tissue cells, demonstrating a high degree of tumor association of the source genes. Moreover, the peptides themselves are presented on tumor tissue. "Tumor tissue" in relation to this invention shall mean a sample from a patient suffering from hematologic neoplasm, such as chronic lymphocytic leukemia, chronic myeloid leukemia or acute myeloid leukemia. Exemplary "full-length polypeptides" or source proteins are indicated in Table 1, column "Protein" or "Protein name", respectively.

In an embodiment of the invention the peptide is isolated. The term "isolated" means that the material is removed from its original environment (e.g., the natural environment, if it is naturally occurring). For example, a naturally-occurring polynucleotide or polypeptide present in a living animal is not isolated, but the same polynucleotide or polypeptide, separated from some or all of the coexisting materials in the natural system, is isolated. Such polynucleotides could be part of a vector and/or such polynucleotides or polypeptides could be part of a composition, and still be isolated in that such vector or composition is not part of its natural environment.

The peptides and/or nucleotides disclosed in accordance with the present invention may also be in "purified" form. The term "purified" does not require absolute purity; rather, it is intended as a relative definition, and can include preparations that are highly purified or preparations that are only partially purified, as those terms are understood by those of skill in the relevant art. For example, individual clones isolated from a cDNA library have been conventionally purified to electrophoretic homogeneity. Purification of starting material or natural material to at least one order of magnitude, preferably two or three orders, and more preferably four or five orders of magnitude is expressly contemplated. Furthermore, a claimed polypeptide which has a purity of preferably 99.999%, or at least 99.99% or 99.9%; and even desirably 99% by weight or greater is expressly encompassed.

The peptides and nucleic acids according to the invention may be in "enriched form". As used herein, the term "enriched" means that the concentration of the material is at least about 2, 5, 10, 100, or 1000 times its natural concentration (for example), advantageously 0.01%, by weight, preferably at least about 0.1% by weight. Enriched preparations of about 0.5%, 1%, 5%, 10%, and 20% by weight are also contemplated. The sequences, constructs, vectors, clones, and other materials comprising the present invention can advantageously be in enriched or isolated form.

The problem underlying the invention is herewith completely solved.

In a particularly preferred embodiment of the invention the peptide consists or consists essentially of an amino acid sequence according to SEQ ID NO: 1 to SEQ ID NO: 20.

"Consisting essentially of" shall mean that a peptide according to the present invention, in addition to the sequence according to any of SEQ ID NO: 1 to SEQ ID NO 20: or a variant thereof contains additional N- and/or C-terminally located stretches of amino acids that are not necessarily forming part of the peptide that functions as an epitope for MHC molecules epitope.

Nevertheless, these stretches can be important to provide an efficient introduction of the peptide according to the present invention into the cells. In one embodiment of the present invention, the peptide is part of a fusion protein which comprises, for example, N- or C-terminal amino acids. In other fusions, the peptides of the present invention can be fused to an antibody as described herein, or a functional part thereof, in particular into a sequence of an antibody, so as to be specifically targeted by said antibody, or, for example, to or into an antibody that is specific for dendritic cells as described herein.

In addition, the peptide or variant may be modified further to improve stability and/or binding to MHC molecules in order to elicit a stronger immune response. Methods for such an optimization of a peptide sequence are well known in the art and include, for example, the introduction of reverse peptide bonds or non-peptide bonds.

In an embodiment of the invention said peptide has the ability to bind to an MHC class I or II molecule, and wherein said peptide, when bound to said MHC, is capable of being recognized by CD4 and/or CD8 T cells.

This measure has the advantage that the capability of the peptide according to the invention to induce an immune response, in particular a T cell response, is ensured.

In another embodiment of the invention the amino acid sequence of the peptide or variant thereof according to the invention comprises a continuous stretch of amino acids of any one of SEQ ID NO: 1 to SEQ ID NO: 20.

This measure has the advantage that the peptide or variant thereof according to the invention comprises all amino acids which are predicted as being involved in the induction of an immune response. The therapeutic efficacy is herewith further improved.

Another subject-matter of the present invention relates to an antibody, in particular a soluble or membrane-bound antibody, preferably a monoclonal antibody or fragment thereof, which specifically recognizes the peptide or variant thereof according to the invention, preferably when bound to an MHC molecule.

The term "antibody" or "antibodies" is used herein in a broad sense and includes both polyclonal and monoclonal antibodies. In addition to intact or "full" immunoglobulin molecules, also included in the term "antibodies" are fragments (e.g. CDRs, Fv, Fab and Fc fragments) or polymers of those immunoglobulin molecules and humanized versions of immunoglobulin molecules, as long as they exhibit any of the desired properties, i.e. specifically recognize the peptide or variant thereof according to the invention. Whenever possible, the antibodies of the invention may be purchased from commercial sources. The antibodies of the invention may also be generated using well-known methods.

The features, characteristics, advantages and embodiments disclosed for the peptide according to the invention apply to the antibody and fragment thereof correspondingly.

Another subject-matter of the invention relates to a T cell receptor, preferably soluble or membrane-bound, or a fragment thereof, that is reactive with an HLA ligand, wherein said ligand is the peptide or variant thereof according to the invention, preferably when bound to an MHC molecule.

The term "T cell receptor" (abbreviated TCR) according to the invention refers to a heterodimeric molecule comprising an alpha polypeptide chain (alpha chain) and a beta polypeptide chain (beta chain), wherein the heterodimeric receptor is capable of binding to a peptide antigen presented by an HLA molecule. The term also includes so-called gamma/delta TCRs.

The features, characteristics, advantages and embodiments disclosed for the peptide and antibody or fragment thereof according to the invention apply to the T cell receptor correspondingly.

A still further subject-matter according to the invention relates to a nucleic acid, encoding for a peptide or variant thereof according to the invention, an antibody or fragment thereof according to the invention, a T cell receptor or fragment thereof according to the invention, optionally linked to a heterologous promoter sequence, or an expression vector expressing said nucleic acid.

The nucleic acid coding for a particular peptide, oligopeptide, or polypeptide may be naturally occurring or they may be synthetically constructed. The nucleic acid (for example a polynucleotide) may be, for example, DNA, cDNA, PNA, RNA or combinations thereof, either single- and/or double- stranded, or native or stabilized forms of polynucleotides, such as, for example, polynucleotides with a phosphorothioate backbone and it may or may not contain introns so long as it codes for the peptide. Of course, only peptides that contain naturally occurring amino acid residues joined by naturally occurring peptide bonds are encodable by a polynucleotide. A still further aspect of the invention provides an expression vector capable of expressing a polypeptide according to the invention.

As used herein the term "nucleic acid coding for (or encoding) a peptide" refers to a nucleotide sequence coding for the peptide including artificial (man-made) start and stop codons compatible for the biological system the sequence is to be expressed by, for example, a dendritic cell or another cell system useful for the production of TCRs. The term "promoter" means a region of DNA involved in binding of RNA polymerase to initiate transcription.

In an embodiment of the invention the nucleic acid is isolated. The term "isolated" means that the material is removed from its original environment (e.g., the natural environment, if it is naturally occurring). For example, a naturally-occurring polynucleotide or polypeptide present in a living animal is not isolated, but the same polynucleotide or polypeptide, separated from some or all of the coexisting materials in the natural system, is isolated. Such polynucleotides could be part of a vector and/or such polynucleotides or polypeptides could be part of a composition, and still be isolated in that such vector or composition is not part of its natural environment.

The features, characteristics, advantages and embodiments disclosed for the peptide according to the invention apply to the nucleic acid and expression vector correspondingly.

Another subject-matter of the invention relates to a recombinant host cell comprising the peptide according to the invention, the antibody or fragment thereof according to the invention, the T cell receptor or fragment thereof according to the invention or the nucleic acid or the expression vector according to the invention, wherein said host cell preferably is selected from a mammalian or human cell, further preferably from an antigen presenting cell, such as a dendritic cell, a T cell or an NK cell.

The features, characteristics, advantages and embodiments disclosed for the peptide according to the invention apply to the host cell correspondingly.

A still further subject-matter of the invention relates to an *in vitro* method for producing activated T lymphocytes, the method comprising contacting in vitro T cells with antigen loaded human class I or II MHC molecules expressed on the surface of a suitable antigen-presenting cell or an artificial construct mimicking an antigen-presenting cell for a period of time sufficient to activate said T cells in an antigen specific manner, wherein said antigen is a peptide according to the invention.

The activated T cells that are directed against the peptides of the invention are useful in therapy. Thus, a further aspect of the invention provides activated T cells obtainable by the foregoing methods of the invention.

Activated T cells, which are produced by the above method, will selectively recognize a cell that aberrantly expresses a polypeptide that comprises an amino acid sequence of SEQ ID NO: 1 to SEQ ID NO: 20.

The features, characteristics, advantages and embodiments disclosed for the peptide according to the invention apply to the above method correspondingly.

Another subject-matter according to the invention relates to a pharmaceutical composition comprising at least one active ingredient selected from the group consisting of the peptide according to the invention, the antibody or fragment thereof according to the invention, the T cell receptor or fragment thereof according to the invention, the nucleic acid or the expression vector according to the invention, the recombinant host cell according to the invention, or the activated T lymphocyte according to the invention, or a conjugated or labelled active ingredient, and a pharmaceutically acceptable carrier, and optionally, pharmaceutically acceptable excipients and/or stabilizers.

A "pharmaceutical composition" is a composition suitable for administration to a human being in a medical setting. Preferably, a pharmaceutical composition is sterile and produced according to GMP guidelines.

The pharmaceutical compositions comprise the peptides either in the free form or in the form of a pharmaceutically acceptable salt (see also above). As used herein, "a pharmaceutically acceptable salt" refers to a derivative of the disclosed peptides wherein the peptide is modified by making acid or base salts of the agent. For example, acid salts are prepared from the free base (typically wherein the neutral form of the drug has a neutral -NH₂ group) involving reaction with a suitable acid. Suitable acids for preparing acid salts include both organic acids, e.g., acetic acid, propionic acid, glycolic acid, pyruvic acid, oxalic acid, malic acid, malonic acid, succinic acid, maleic acid, fumaric acid, tartaric acid, citric acid, benzoic acid, cinnamic acid, mandelic acid, methane sulfonic acid, ethane sulfonic acid, p-toluenesulfonic acid, salicylic acid, and the like, as well as inorganic acids, e.g., hydrochloric acid, hydrobromic acid, sulfuric acid, nitric acid phosphoric acid and the like. Conversely, preparation of basic salts of acid moieties which may be present on a peptide are prepared using a pharmaceutically acceptable base such as sodium hydroxide, potassium hydroxide, ammonium hydroxide, calcium hydroxide, trimethylamine or the like.

Preferably, the pharmaceutical composition of the present invention is an immunotherapeutic such as a vaccine. It may be administered directly into the patient, into the affected organ or systemically i.d., i.m., s.c., i.p. and i.v., or applied ex *vivo* to cells derived from the patient or a human cell line which are subsequently administered to the patient, or used *in vitro* to select a subpopulation of immune cells derived from the patient, which are then re-administered to the patient. If the nucleic acid is administered to cells *in vitro,* it may be useful for the cells to be transfected so as to co-express immune-stimulating cytokines, such as interleukin-2.

The peptide may be substantially pure. The peptide may also be combined with an immune-stimulating adjuvant, such as the TLR1/2 ligand XS15. Preliminary work has shown that XS15 can induce a strong CD8⁺ and Th1CD4⁺ T-cell response *in vivo* after subcutaneous injection in healthy donors and individual tumor patients for viral, mutated and unmutated peptides in a water-oil emulsion. The peptide may also be used in combination with immune-stimulatory cytokines, or be administered with a suitable delivery system, for example liposomes. The peptide may also be conjugated to a suitable carrier such as keyhole limpet haemocyanin (KLH) or mannan (see WO 95/18145 and (Longenecker et al., Ann. N.Y. Acad. Sci. 690:276-291 (1993)). The peptide may also be tagged, may be a fusion protein, or may be a hybrid molecule. The peptides whose sequence is given in the present invention are expected to stimulate CD4 or CD8 T cells. However, stimulation of CD8 T cells is more efficient in the presence of help provided by CD4 T-helper cells. Thus, for MHC Class I epitopes that stimulate CD8 T cells the fusion partner or sections of a hybrid molecule suitably provide epitopes which stimulate CD4-positive T cells. CD4- and CD8-stimulating epitopes are well known in the art and include those identified in the present invention. In one aspect, the vaccine comprises at least one peptide having the amino acid sequence set forth SEQ ID NO: 1 to SEQ ID NO: 20, and at least one additional peptide, preferably two to 50, more preferably two to 25, even more preferably two to 20 and most preferably two, three, four, five, six, seven, eight, nine, ten, eleven, twelve, thirteen, fourteen, fifteen, sixteen, seventeen or eighteen peptides. The peptide(s) may be derived from one or more specific TAAs and may bind to MHC class I molecules.

In an embodiment of the pharmaceutical composition according to the invention it comprises at least 5, preferably at least 6, further preferably at least 7, further preferably at least 8, further preferably at least 9, and highly preferably at least 10 different peptides, each peptide comprising an amino acid sequence selected from the group consisting of SEQ ID NO: 1 to SEQ ID NO: 20, and variant sequences thereof which are at least 88% homologous to SEQ ID NO: 1 to SEQ ID NO: 20, and wherein said variant binds to molecule(s) of the major histocompatibility complex (MHC) and/or induces T cells cross-reacting with said variant peptide; and a pharmaceutical acceptable salt thereof, wherein said peptide is not a full-length polypeptide.

The inventors have realized that a pharmaceutical composition, such as a vaccine, has a good immunogenic effect if at least 5 peptides or amino acid sequences out of the SEQ ID NOS. 1 to 20 are contained therein. When using 10 peptides or amino acid sequences a very high percentage of the human world population may be covered. Preferably the vaccine comprises 1 to 6 peptides comprising amino acid sequences out of the SEQ ID NOS. 1 to 6, and 1 to 9 peptides comprising amino acid sequences out of the SEQ ID NOS. 7 to 20. "1 to 6" in this context means 1, 2, 3, 4, 5, or 6. "1 to 9" in this context means 1, 2, 3, 4, 5, 6, 7, 8, or 9. This measure has the advantage that it is ensured that at least one MHC class II and at least one MHC class I peptide is used.

In this context it is a preferred embodiment of the pharmaceutical composition if the at least 1 peptide or amino acid sequence is selected out of each of the following groups: SEQ ID NOs: 1 to 6; SEQ ID NOs: 7 to 13; SEQ ID NOs: 14 to 17; SEQ ID NOs: 18 to 20. "At least 1" means 1, 2, 3, 4, 5, 6. This ensures coverage of a very high percentage of the world population.

The pharmaceutical composition according to the invention may comprise, as the only active ingredient, the peptide according to the invention, the antibody or fragment thereof according to the invention, the T cell receptor or fragment thereof according to the invention, the nucleic acid or the expression vector according to the invention, the recombinant host cell according to the invention, or the activated T lymphocyte according to the invention. However, in an alternative aspect, it may comprise an additional active ingredient such as a small molecule, e.g. ibrutinib and/or ideasilib and/or venetoclax.

The features, characteristics, advantages and embodiments disclosed for the peptide according to the invention apply to the pharmaceutical composition correspondingly.

Another subject-matter of the invention relates to the peptide according to the invention, the antibody or fragment thereof according to the invention, the T cell receptor or fragment thereof according to the invention, the nucleic acid or the expression vector according to the invention, the recombinant host cell according to the invention, or the activated T lymphocyte according to the invention for use in medicine, preferably in the diagnosis and/or prevention and/or treatment of cancer, or for use in the manufacture of a medicament against cancer.

In an embodiment of the invention said cancer is selected from the group of chronic lymphocytic leukemia (CLL), acute myeloid leukemia (AML), chronic myeloid leukemia (CML) and other hematologic and/or lymphoid neoplasms, for example, Non-Hodgkin lymphoma, post-transplant lymphoproliferative disorders (PTLD) as well as other myeloid neoplasms, such as primary myelofibrosis, essential thrombocytopenia, polycythemia vera, as well as other neoplasms such as hepatocellular carcinoma, colorectal carcinoma, glioblastoma, gastric cancer, oesophageal cancer, non-small cell lung cancer, small cell lung cancer, pancreatic cancer, renal cell carcinoma, prostate cancer, melanoma, breast cancer, gallbladder cancer and cholangiocarcinoma, urinary bladder cancer, uterine cancer, head and neck squamous cell carcinoma, mesothelioma and other tumors that show an overexpression of a protein from which a peptide SEQ ID NO: 1 to SEQ ID NO: 20 is derived from.

The features, characteristics, advantages and embodiments disclosed for the peptide according to the invention apply to the above-referenced use correspondingly.

A still further subject-matter according to the invention relates to a kit comprising:
a) a container comprising a pharmaceutical composition containing the peptide(s) or the variant according to the invention, the antibody or fragment thereof according to the invention, the T cell receptor or fragment thereof according to the invention, the nucleic acid or the expression vector according to the invention, the recombinant host cell according to the invention, or the activated T lymphocyte according to the invention, in solution or in lyophilized form;
b) optionally, a second container containing a diluent or reconstituting solution for the lyophilized formulation;
c) optionally, at least one more peptide selected from the group consisting of SEQ ID NO: 1 to SEQ ID NO: 20, and
d) optionally, instructions for (i) use of the solution or (ii) reconstitution and/or use of the lyophilized formulation.

The kit may further comprise one or more of (iii) a buffer, (iv) a diluent, (v) a filter, (vi) a needle, or (v) a syringe. The container is preferably a bottle, a vial, a syringe or test tube; and it may be a multi-use container. The pharmaceutical composition is preferably lyophilized.

The kits of the present invention preferably comprises a lyophilized formulation of the present invention in a suitable container and instructions for its reconstitution and/or use. Suitable containers include, for example, bottles, vials (e.g. dual chamber vials), syringes (such as dual chamber syringes) and test tubes. The container may be formed from a variety of materials such as glass or plastic. Preferably the kit and/or container contain/s instructions on or associated with the container that indicates directions for reconstitution and/or use. For example, the label may indicate that the lyophilized formulation is to be reconstituted to peptide concentrations as described above. The label may further indicate that the formulation is useful or intended for subcutaneous administration.

The features, characteristics, advantages and embodiments disclosed for the peptide according to the invention apply to the kit correspondingly.

Another subject-matter of the invention relates to a method for producing a personalized anti-cancer pharmaceutical composition, preferably a vaccine, said method comprising:
a) identifying tumor-associated peptides (TUMAPs) presented by a tumor sample from an individual patient;
b) comparing the peptides as identified in step a) with a warehouse of peptides which have been pre-screened for immunogenicity and/or over-presentation in tumors as compared to normal tissues;
c) selecting at least one peptide from the warehouse that matched a TUMAP identified in said patient; and
d) formulating the personalized vaccine based on step c),
wherein said warehouse comprises a plurality of peptides and/or variant sequences according to the invention.

A "personalized pharmaceutical composition" shall mean specifically tailored therapies for one individual patient that will only be used for therapy in such individual patient, including actively personalized cancer vaccines and adoptive cellular therapies using autologous patient tissue.

As used herein, the term "warehouse" shall refer to the peptides according to the invention that have been pre-screened for immunogenicity and/or over-presentation in a particular tumor type, in particular hematologic neoplasms, such as CLL. The warehouse (e.g. in the form of a data-base) is composed of tumor-associated peptides which were highly overexpressed in cancer cells of patients with various HLA-A HLA-B and HLA-C alleles. It contains MHC class I and MHC class II peptides. In particular, it contains MHC class I A*24, B*07, and A*02 marker peptides. These peptides allow comparison of the magnitude of T-cell immunity induced by TUMAPS in a quantitative manner and hence allow important conclusion to be drawn on the capacity of the vaccine to elicit anti-tumor responses.

In an embodiment, the HLA phenotype is gathered from the patients' tumor material. In case it turned out that the patient exhibits the HLA type A*24 the pharmaceutical composition or vaccine will include any or all of the peptides comprising any of SEQ ID NOs: 7 to 13. In case of HLA type B*07 it will include any or all of the peptides comprising any of SEQ ID NOs: 14 to 17. In case of HLA type A*02 it will include any or all of the peptides comprising any of SEQ ID NOs: 18 to 20.

In one exemplary embodiment, the peptides included in the vaccine are identified by: (a) identifying tumor-associated peptides (TUMAPs) presented by a tumor sample from the individual patient by the method as described above; (b) comparing the peptides identified in a) with the warehouse of peptides that have been prescreened for immunogenicity and overpresentation in tumors as compared to corresponding normal tissue; (c) selecting at least one peptide from the warehouse that correlates with a tumor-associated peptide identified in the patient; and (d) optionally, selecting at least one peptide identified de novo in (a) confirming its immunogenicity.

Once the peptides for a personalized peptide based pharmaceutical composition, e.g. a vaccine, are selected, the composition is produced. The composition or vaccine preferably is a liquid formulation consisting of the individual peptides dissolved in between 20-40% DMSO, preferably about 30-35% DMSO, such as about 33% DMSO.

The features, characteristics, advantages and embodiments disclosed for the peptide according to the invention apply to the afore-referenced method correspondingly.

It is to be understood that the before-mentioned features and those to be mentioned in the following cannot only be used in the combination indicated in the respective case, but also in other combinations or in an isolated manner without departing from the scope of the invention.

### EMBODIMENTS

The invention is now further explained by means of embodiments resulting in additional features, characteristics and advantages of the invention. The embodiments are of pure illustrative nature and do not limit the scope or range of the invention. The features mentioned in the specific embodiments are features of the invention and may be seen as general features which are not applicable in the specific embodiment but also in an isolated manner in the context of any embodiment of the invention.

The invention is now further described and explained in further detail by referring to the following non-limiting examples and figures:

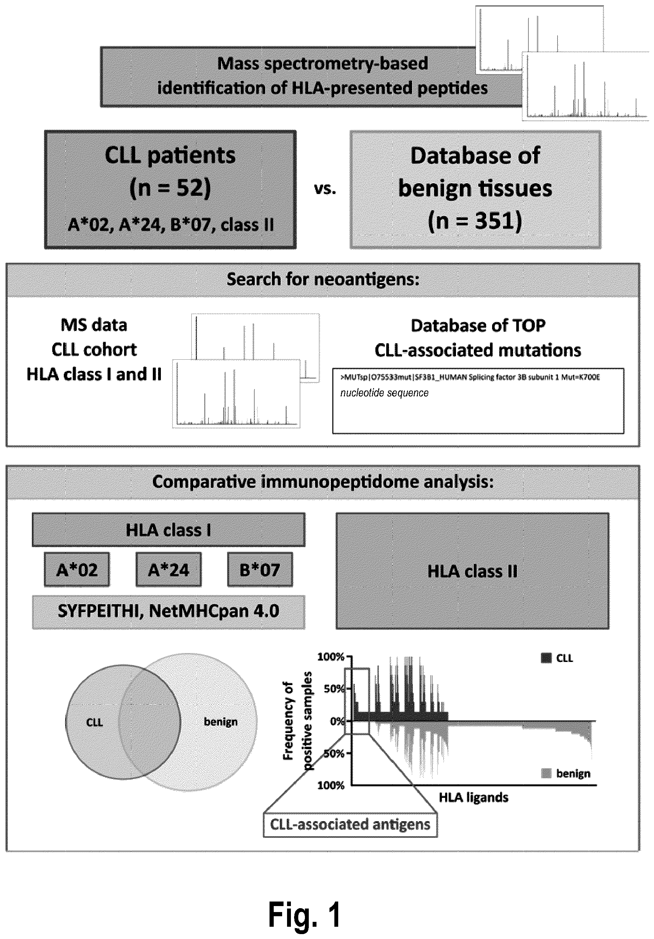

- Fig. 1:: Schematic overview of warehouse peptide selection workflow by mass spectrometric HLA ligandome analysis of primary CLL cells, screening for mutation-derived HLA-presented peptides and comparative immunopeptidome analysis with a large benign tissue-derived HLA ligandome database.

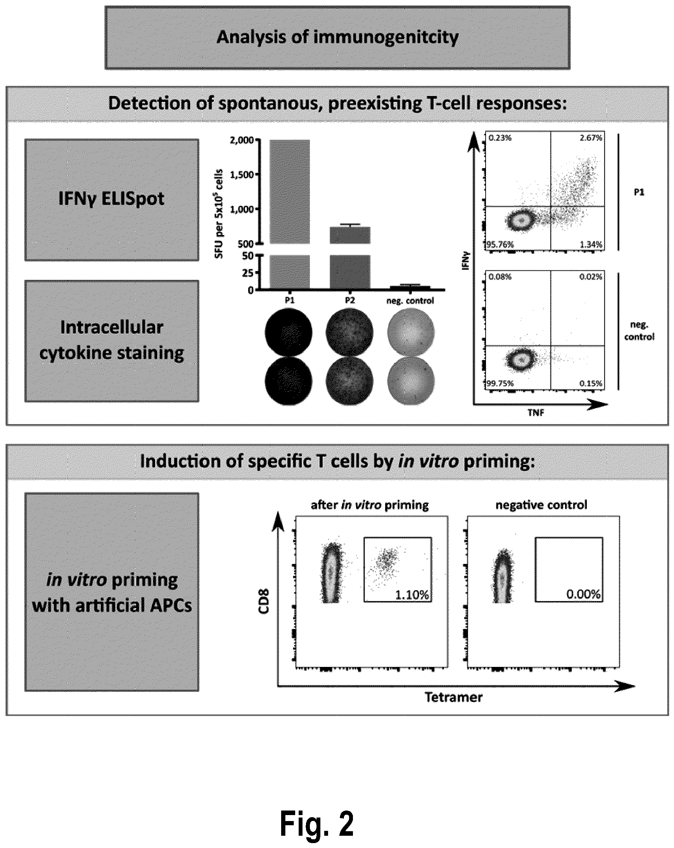

- Fig. 2:: Schematic overview of immunogenicity analyses of warehouse peptides based on the detection of spontaneous peptide-specific T cell responses via IFN-γ ELISPOT and intracellular cytokine staining or by in vitro priming using T cells of healthy volunteer and CLL patients by mass spectrometric HLA ligandome analysis of primary CLL cells, screening for mutation-derived HLA-presented peptides and comparative immunopeptidome analysis with a large benign tissue-derived HLA ligandome data-base.

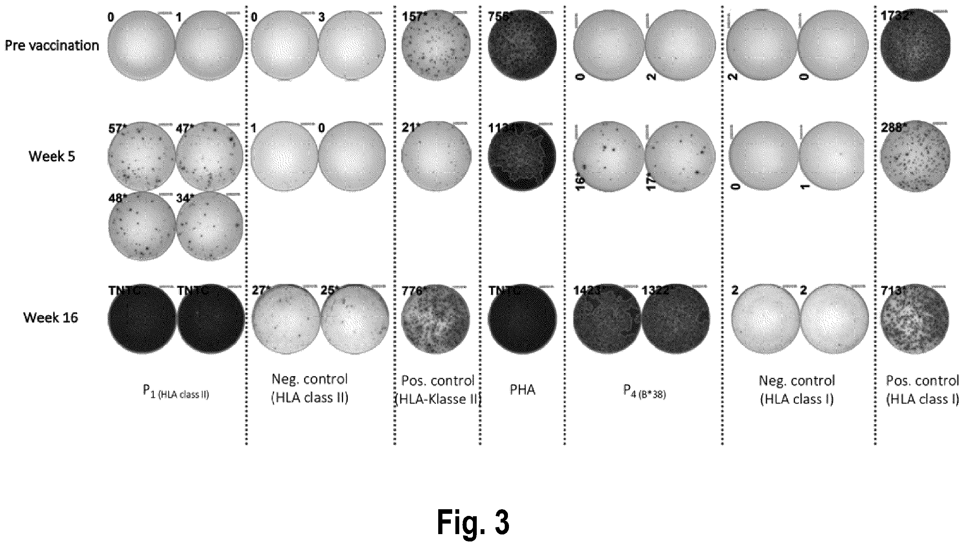

- Fig. 3:: INF-γ ELISPOT immunomonitoring of a colon carcinoma patient before subcutaneous personalized peptide vaccination with XS15 in montanide 5 weeks and 16 weeks after vaccination. The functionality of peptide-specific CD4+ and CD8+ T cells for two vaccine cocktail peptides is demonstrated. A total of two vaccinations (week 1 and week 8) were carried out as part of an individual healing trial. The selection of the peptides was based on a mutation panel analysis and an individual HLA ligandome analysis of the patient's tumor tissue.

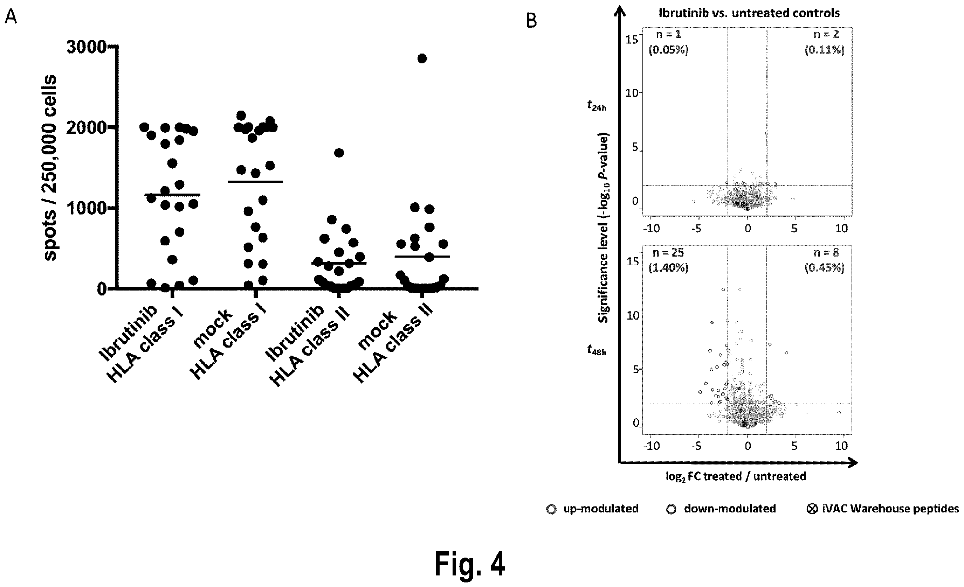

- Fig. 4:: Influence of the BTK inhibitor ibrutinib on (A) peptide-induced T cell response and (B) the HLA ligandome of CLL. (A) Retrospective analysis of preexisting immune responses directed against HLA class I and HLA class II viral T cell epitopes analyzed in IFN-γ ELISPOT assays after 12-d recall stimulation of PBMCs from CLL patients treated in vitro with ibrutinib or mock control. (B) Volcano plots of immunopeptidome modulations in the relative abundances of HLA ligands on primary CLL cells treated in vitro with ibrutinib versus mock treated cells at t24h and t48h, respectively. Each dot represents a specific HLA ligand. Log2-fold-changes of their abundance are indicated on the x-axis, the corresponding significance levels on the y-axis. HLA ligands showing significant up-or down-modulation are highlighted in red and blue. CLL warehouse peptides are marked in black.

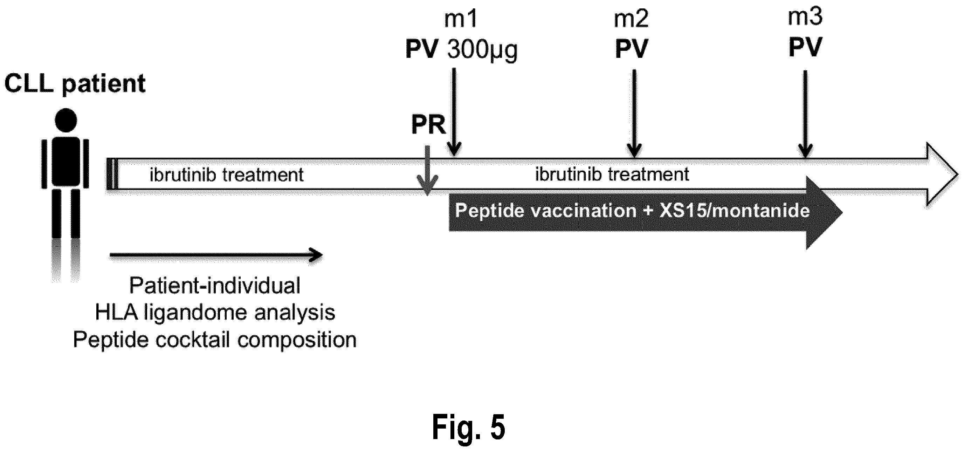

- Fig. 5:: Study design.

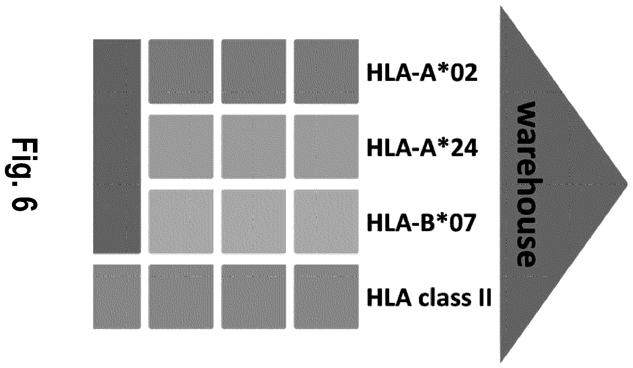

- Fig. 6:: CLL peptide warehouse.

### 1. Material and Methods

### Patient samples

For HLA ligandome analysis, peripheral blood monocular cells from CLL patients were collected at the Department of Hematology and Oncology in Tübingen. Cells were isolated by density gradient centrifugation and stored at 80°C until further use. Informed consent was obtained in accordance with the Declaration of Helsinki protocol. The study was performed according to the guidelines of the local ethics committees (373/2011B02, 454/2016B02). HLA typing was carried out by the Department of Hematology and Oncology, Tübingen, Germany.

### Isolation of HLA ligands

HLA class I and II molecules were isolated by standard immunoaffinity purification using the pan-HLA class I-specific W6/32 monoclonal antibody, the pan-HLA class II-specific Tü-39 monoclonal antibody, and the HLA-DR-specific L243 monoclonal antibody to extract HLA ligands.

### Analysis of HLA ligands by LC-MS/MS

Peptides of HLA ligand extracts were separated by nanoflow high-performance liquid chromatography (RSLCnano, Thermo Fisher Scientific) using a 50 µm x 25 cm PepMap rapid separation liquid chromatography column and a gradient ranging from 2.4% to 32.0% acetonitrile over the course of 90 min. Eluting peptides were analyzed in an online-coupled LTQ Orbitrap Fusion Lumos mass spectrometer (Thermo Fisher Scientific) equipped with a nanoelectron spray ion source using a data dependent acquisition mode employing a top speed CID fragmentation method (normalized collision energy 35%). Mass range for HLA class I peptide analysis was set to 400 - 650 m/z with charge states 2+ and 3+ selected for fragmentation. For HLA class II peptide analysis mass range was limited to 400 - 1,000 m/z with charge states 2+ to 5+ selected for fragmentation.

### Data processing and HLA annotation

For data processing the SEQUEST HT search engine was used to search the human proteome as comprised in the Swiss-Prot database (20,279 reviewed protein sequences, September 27, 2013) without enzymatic restriction. Precursor mass tolerance was set to 5 ppm, and fragment mass tolerance to 0.02 Da. Oxidized methionine was allowed as a dynamic modification. The false discovery rate was estimated using the Percolator algorithm and limited to 5% for HLA class I and 1% for HLA class II. Peptide lengths were limited to 8 - 12 amino acids for HLA class I and to 8 - 25 amino acids for HLA class II. Protein inference was disabled, allowing for multiple protein annotations of peptides. HLA class I annotation was performed using NetMHCpan 4.0 and SYFPEITHI annotating peptides with percentile rank below 2% and ≥ 60% of the maximal score, respectively.

### Amplification of peptide-specific T cells and IFN-γELISPOT assay

Peripheral blood mononuclear cells (PBMCs) were pulsed with 1 µg/mL (class I) or 5 µg/mL(class II) per peptide and cultured for 12 days adding 20 U/mL IL-2 on days 3, 5, and 7. Peptide-stimulated PBMCs were analyzed by ELISPOT assay on day 12.

### Priming of naïve CD8⁺ T cells

Priming of peptide-specific CD8+ T cells was conducted using artificial antigen-presenting cells. Therefore, MACS-sorted CD8+ T cells were cultured with IL 2 and IL-7. Weekly stimulation with peptide-loaded artificial antigen-presenting cells and IL 12 was performed four times.

### Cytokine staining

For intracellular cytokine staining, cells were pulsed with 10 µg/mLof individual peptide and incubated with 10 µg/mLBrefeldin A and 10 µg/mLGolgiStop for 12 - 16 h. Staining was performed using Cytofix/Cytoperm, PerCP anti-human CD8, Pacific Blue anti-human TNF, FITC anti-human CD107a, and PE anti-human IFN-γ monoclonal antibodies. PMA and ionomycin served as positive control. Samples were analyzed on a FACS Canto II cytometer.

### Tetramer staining

The frequency of peptide-specific CD8+ T cells after priming was determined by PE/Cy7 anti-human CD8 monoclonal antibody and HLA:peptide tetramer-PE staining. Tetramers of the same HLA allotype containing irrelevant control peptides were used as negative control. Samples were analyzed on a FACS Canto II cytometer.

### 2. Results

### In vitro experiments

Personalized peptide vaccine cocktails are based on the individualized HLA ligandome analysis of the CLL cells of each study patients. To allow for a timely and cost-efficient production of personalized vaccine cocktails for a large patient study cohort the inventors developed a so called "warehouse" concept. This premanufactured peptide warehouse includes, based on inventors' preclinical data, the most frequent and CLL-associated antigens for the most common HLA allotypes.

Vaccine cocktail composition for each individual patient is than performed based on the results of the HLA ligandome analysis of CLL, providing the proof for the natural presentation of warehouse peptides on CLL cells. The feasibility of this warehouse approach was approved by the first results of an ongoing study (Kowalewski et al. (2014), *I.c*.), showing for more than 90% of the study patient at least on naturally presented warehouse peptide in the immunopeptidome of CLL cells. Therefore, this warehouse approach will be also applied in a study further demonstrating the benefits of the invention in the clinic.

The inventors redesigned the CLL warehouse based on the results of preclinical data on 52 HLA ligandomes of primary CLL cells and the first data of a previous trial using a novel innovative workflow as depicted in Figure 1.

In contrast to analyses in the art where one could detect naturally presented mutation-derived peptides from specific mutations an in-depth search in or mass spectrometric data for mutation-derived peptides for the most common CLL-specific mutations revealed no naturally presented neoepitopes. Therefore, the inventors focused for the design of the peptide warehouse on non-mutated CLL-associated antigens. These were selected based on a direct comparative immunopeptidome analysis using a proprietary large in-house database including more than 350 immunopeptidomes of various benign tissues including B cells, bone marrow, CD34⁺ progenitor cells as well as solid organ tissues. Based on the data of an ongoing clinical study the inventors selected A*02, A*24 and B*07 as most common HLA allotypes in the CLL cohort for the novel peptide warehouse. For these HLA class I allotypes as well as for HLA class II the inventors selected the three and five most frequent CLL-exclusive antigens for the peptide warehouse, respectively.

With this approach the Inventors developed the peptides comprising the amino acid sequences SEQ ID NOs: 1-20, which are depicted in above table 1.

In a next step the potential of the warehouse peptides according to the invention to induced functional T cell responses was proven either by characterizing of spontaneous T cell responses against our peptides in CLL patients using IFN-γ ELISPOT and intracellular cytokine staining or by *in vitro* priming using T cells of healthy volunteer and CLL patients.

A schematic overview of the immunogenicity analyses is depicted in Figure 2.

To induce strong and clinically effective T cell responses in the patient, the combination of peptide-based immunotherapy with appropriate adjuvants is a main prerequisite. So far only the TLR ligand imiquimod locally applied on vaccination side was available for use in clinical peptide vaccination studies. Thus, the inventors characterized in recent years the new water-soluble and thus easily GMP-producible TLR2 ligand XS15. Preliminary work has shown that XS15 can induce a strong CD8⁺ and Th1CD4⁺ T cell response *in vivo* after subcutaneous injection in healthy donors and individual tumor patients for viral, mutated and unmutated peptides in a water-oil emulsion (montanide ISA 51) (Figure 3).

XS15 results in granuloma formation on vaccination site where the vaccinated peptides persist for at least 7 weeks. Furthermore peptide-specific T cells could also be detected on granuloma site, however with a lower frequency than observed in peripheral blood, which confirms that there is no risk of T cell sequestration, dysfunction or deletion on vaccination site using XS15 in montanide. Strikingly, the induced immune responses persist for more than 1.5 years.

Ibrutinib represents a new treatment standard in first- and second-line therapy for CLL. Therefore, in an embodiment the inventors' multi-peptide vaccine may be applied in patients under ibrutinib treatment. As described, several groups have postulated the positive effect of ibrutinib on anti-cancer immune response. However, ibrutinib is also proposed as potential agent to treat graft versus host disease after allogeneic stem cell transplantation, due to its inhibitory effect on Th2 cells. Thus, the inventors evaluated in own preclinical work the effect of ibrutinib on peptide-induced T cell response, showing no difference in the IFN-γ production of CLL patient-derived T cells to an EBV (Epstein-Barr virus) peptide mix with and without in vitro application of ibrutinib (Figure 4A).

Furthermore, the inventors could show that ibrutinib has no relevant effect on the HLA class I and II expression as well as on the composition of the immunopeptidome of CLL cells, warranting the stable presentation of the CLL-associated peptides according to the invention under ibrutinib treatment (Figure 4B).

### Clinical study

The clinical study is an open label phase l/lI study evaluating a personalized multi-peptide vaccination in combination with the TLR2 ligand XS15 in CLL patients under ibrutinib treatment. The primary objective of this trial is to evaluate the safety and tolerability of the individually composed multi-peptide vaccine in combination with the TLR2 agonist XS15 in montanide ISA51. Toxicity is determined by evaluation of the number of adverse events according to CTCAE V 5.0. The secondary objective of this trial is to evaluate the immunogenicity of the individually composed multi-peptide vaccine in combination with the adjuvant XS15 as determined by induction of T cell immunity. The induction of peptide-specific T-cell responses will be determined by IFN-γ ELISPOT assays.

These assays are performed after obtaining and processing of the last blood sample of each individual patient one month (end of treatment visit) and seven months (follow up) after the last peptide vaccination. Further secondary objectives of this trial are progression free survival (PFS) and the remission status as well as the evaluation of achievement of MRD-negativity or MRD level reduction at the end of study. Furthermore, for all patients participating in this study PFS, overall survival (OS) and remission status will be assessed two and five years after the end of the study outside the study protocol. In further exploratory endpoints the inventors will correlate the inducibility of immune responses to the multi-peptide vaccine with clinical, biological and mass spectrometric patient characteristics to develop novel biomarkers predicting the immunological and clinical response to peptide-based immunotherapy.

Physically fit patients (as defined by ECOG score ≤2) with a confirmed diagnosis of a CLL or small lymphocytic lymphoma (SLL) according to the IWCLL guidelines who have achieved at least a partial remission (PR) (IWCLL guidelines for the diagnosis and treatment of CLL) under ibrutinib treatment but are still MRD positive (CLL cells in peripheral blood or bone marrow ≥ 10⁻⁴, determined by flow cytometry) are treated within the study.

Study inclusion (screening visit) is performed before the start of ibrutinib treatment, to allow for an analysis of individual patients' immunopeptidome of CLL cells. CLL patients included in the study receive ibrutinib as a mono therapy, no combination with chemotherapy, antibody therapy or other small molecules is allowed. Patients included in the study can receive ibrutinib as first line treatment or at disease relapse. Previous CLL specific therapies are assessed during the screening visit. Peptide vaccination takes place in CLL patients that achieved at least a partial remission with detectable MRD after at least two and less than 12 months of ibrutinib treatment. Vaccination takes place every four weeks. A total of three vaccinations are carried out. Vaccines are applied subcutaneously in the abdomen after emulsification of the vaccine cocktail (10 peptides + XS15) in montanide ISA51. Ibrutinib treatment will be continued during peptide vaccination and afterwards (Figure 5).

### Warehouse production and peptide cocktail formulation

Multi-peptide vaccine cocktails consist, in an embodiment, of 300 µg of each of CLL-associated peptides. In an embodiment the vaccine cocktail consists of three HLA class I and five HLA class II-restricted CLL-associated peptides as well as one HLA class II-restricted adenovirus (ADV) and one HLA-class II-restricted survivin-derived control peptide. The HLA class I-restricted peptides for each patient are selected individually based on the patient-individual HLA allotypes and immunopeptidome analysis of CLL cells from a premanufactured warehouse consisting of, in an embodiment, 9 different peptides restricted to the 3 most common HLA class I allotypes observed in the CLL patient cohort (A*02, A*24, B*07). Peptides were synthesized in the GMP-certified Wirk-stoffpeptidlabor at the University of Tübingen. All peptides are synthetic peptides, which are manufactured by well-established solid phase peptide synthesis (SPPS) procedures using Fmoc chemistry.

Vaccine cocktail formulation (10 patient-individual warehouse peptides and the TLR-2 ligand XS15 (0,5mg/ml) in H₂O/DMSO) is performed at the GMP-Center of the University Hospital Tübingen.

The warehouse peptides comprise the most frequently detected CLL-associated antigens identified in the inventors' experimental and clinical studies, as described above. HLA class I peptides to be applied in each CLL patient are selected in a personalized manner guided by the mass spectrometric analysis of the patient-individual HLA ligandome. Therefore, LC/MS/MS-based immunopeptidome analyses are performed from CLL cells, taken at the screening visit before the start of ibrutinib treatment. In addition, in an embodiment, seven HLA class II peptides (five CLL-associated warehouse peptides and two control peptides) are be administered in every patient (Figure 6).

### 3. Conclusion

The inventors were able to develop peptides which do allow the production of a personalized multi-peptide vaccine against cancer, e.g. hematologic neoplasms such as CLL, a cancer entity mainly affecting elderly patients. The approach according to the invention overcomes the shortcomings in the art, for the following reasons:
- Peptide cocktail selection from a premanufacture warehouse will allow for a time and cost saving and therefore broadly applicable individualized vaccine production.
- The selection of the warehouse peptides is based on immunopeptidome data from a uniquely large cohort of primary CLL samples (n=52) and the largest immunopeptidome database of healthy tissue (n=350).
- The patient-individual selection of peptide vaccines is based on a direct immunopeptidome analysis of each study patients' CLL cells.
- The invention is accessible to novel adjuvants, such as the TLR1/2 ligand XS15. This will allow for the induction of long-lasting peptide-specific T cell responses, as already proven in preclinical and first clinical evaluations.
- Peptide vaccination can be combined with the standard treatment, such as ibrutinib, for which positive effects on T cell response was reported and does not affect the presentation of CLL-associated peptide targets.
- Peptide vaccination can be applied after reduction of leukemia burden in the MRD setting enabling an optimal effector T cell to target cell ratio.

These particularities overcome limitations of presently available peptide-based strategies and bring T cell-based immunotherapy to the next level, and to improve treatment options for patients with cancer, in particular hematologic neoplasms such as CLL.

## Claims

1. A peptide comprising an amino acid sequence selected from the group consisting of SEQ ID NO: 1 to SEQ ID NO: 20, and variant sequences thereof which are at least 88% homologous to SEQ ID NO: 1 to SEQ ID NO: 20, and wherein said variant binds to molecule(s) of the major histocompatibility complex (MHC) and/or induces T cells cross-reacting with said variant peptide; and a pharmaceutical acceptable salt thereof, wherein said peptide is not a full-length polypeptide.

2. The peptide according to claim 1, wherein said peptide has the ability to bind to an MHC class I or II molecule, and wherein said peptide, when bound to said MHC, is capable of being recognized by CD4 and/or CD8 T cells.

3. The peptide or variant thereof according to claim 1 or 2, wherein the amino acid sequence thereof comprises a continuous stretch of amino acids according to any one of SEQ ID NO: 1 to SEQ ID NO: 20.

4. An antibody, in particular a soluble or membrane-bound antibody, preferably a monoclonal antibody or fragment thereof, that specifically recognizes the peptide or variant thereof according to any of claims 1 to 3, preferably the peptide or variant thereof according to any of claims 1 to 3 when bound to an MHC molecule.

5. A T cell receptor, preferably soluble or membrane-bound, or a fragment thereof, that is reactive with an HLA ligand, wherein said ligand is the peptide or variant thereof according to any of claims 1 to 3, preferably the peptide or variant thereof according to any of claims 1 to 3 when bound to an MHC molecule.

6. A nucleic acid, encoding for a peptide or variant thereof according to any one of claims 1 to 3, an antibody or fragment thereof according to claim 4, a T cell receptor or fragment thereof according to claim 5, optionally linked to a heterologous promoter sequence, or an expression vector expressing said nucleic acid.

7. A recombinant host cell comprising the peptide according to any one of claims 1 to 3, the antibody or fragment thereof according to claim 4, the T cell receptor or fragment thereof according to claim 5 or the nucleic acid or the expression vector according to claim 6, wherein said host cell preferably is selected from an antigen presenting cell, such as a dendritic cell, a T cell or an NK cell.

8. An *in vitro* method for producing activated T lymphocytes, the method comprising contacting *in vitro* T cells with antigen loaded human class I or II MHC molecules expressed on the surface of a suitable antigen-presenting cell or an artificial construct mimicking an antigen-presenting cell for a period of time sufficient to activate said T cells in an antigen specific manner, wherein said antigen is a peptide according to any one of claims 1 to 3.

9. An activated T lymphocyte, produced by the method according to claim 8, that selectively recognizes a cell which presents a polypeptide comprising an amino acid sequence given in any one of claims 1 to 3.

10. A pharmaceutical composition comprising at least one active ingredient selected from the group consisting of the peptide according to any one of claims 1 to 3, the antibody or fragment thereof according to claim 4, the T-cell receptor or fragment thereof according to claim 5, the nucleic acid or the expression vector according to claim 6, the recombinant host cell according to claim 7, or the activated T lymphocyte according to claim 9, or a conjugated or labelled active ingredient, and a pharmaceutically acceptable carrier, and optionally, pharmaceutically acceptable excipients and/or stabilizers.

11. The pharmaceutical composition according to claim 10, wherein it comprises at least 5, preferably at least 6, further preferably at least 7, further preferably at least 8, further preferably at least 9, and highly preferably at least 10 different peptides, each peptide comprising an amino acid sequence selected from the group consisting of SEQ ID NO: 1 to SEQ ID NO: 20, and variant sequences thereof which are at least 88% homologous to SEQ ID NO: 1 to SEQ ID NO: 20, and wherein said variant binds to molecule(s) of the major histocompatibility complex (MHC) and/or induces T cells cross-reacting with said variant peptide; and a pharmaceutical acceptable salt thereof, wherein said peptide is not a full-length polypeptide.

12. The peptide according to any one of claims 1 to 3, the antibody or fragment thereof according to claim 4, the T cell receptor or fragment thereof according to claim 5, the nucleic acid or the expression vector according to claim 6, the recombinant host cell according to claim 7, or the activated T lymphocyte according to claim 9 for use in medicine, preferably said use is in diagnosis and/or prevention and/or treatment of cancer, or for use in the manufacture of a medicament against cancer, further preferably said cancer is selected from the group consisting of: hematologic neoplasms, chronic lymphocytic leukemia (CLL), acute myeloid leukemia (AML), chronic myeloid leukemia (CML), Non-Hodgkin lymphoma, post-transplant lymphoproliferative disorders (PTLD), myeloid neoplasms, primary myelofibrosis, essential thrombocytopenia, polycythemia vera, hepatocellular carcinoma, colorectal carcinoma, glioblastoma, gastric cancer, oesophageal cancer, non-small cell lung cancer, small cell lung cancer, pancreatic cancer, renal cell carcinoma, prostate cancer, melanoma, breast cancer, gallbladder cancer and cholangiocarcinoma, urinary bladder cancer, uterine cancer, head and neck squamous cell carcinoma, mesothelioma and other tumors that show an overexpression of a protein from which a peptide comprising SEQ ID No. 1 to SEQ ID No. 20 is derived from.

13. The peptide according to claim 12, wherein said medicament is a vaccine.

14. A kit comprising:
(a) a container comprising a pharmaceutical composition containing the peptide(s) or the variant according to any one of claims 1 to 3, the antibody or fragment thereof according to claim 4, the T cell receptor or fragment thereof according to claim 5, the nucleic acid or the expression vector according to claim 6, the recombinant host cell according to claim 7, or the activated T lymphocyte according to claim 9, in solution or in lyophilized form;
(b) optionally, a second container containing a diluent or reconstituting solution for the lyophilized formulation;
(c) optionally, at least one more peptide selected from the group consisting of SEQ ID NO: 1 to SEQ ID NO: 20, and
(d) optionally, instructions for (i) use of the solution or (ii) reconstitution and/or use of the lyophilized formulation.

15. A method for producing a personalized anti-cancer vaccine, said method comprising:
a) identifying tumor-associated peptides (TUMAPs) presented by a tumor sample from an individual patient;
b) comparing the peptides as identified in step a) with a warehouse of peptides which have been pre-screened for immunogenicity and/or over-presentation in tumors as compared to normal tissues;
c) selecting at least one peptide from the warehouse that matched a TUMAP identified in said patient; and
d) formulating the personalized vaccine based on step c),
wherein said warehouse comprises a plurality of peptides and/or variant sequences according to any of claims 1 to 3.


SEQUENCE LISTING

<110>  Eberhard Karls Universität, Medizinische Fakultät

<120>  Peptides and combinations of peptides for use in immunotherapy
       against hematologic neoplasms and other cancer

<130>  5402P605EP

<160>  20

<170>  PatentIn version 3.5

<210>  1
<211>  18
<212>  PRT
<213>  Artificial sequence

<220>
<223>  Tumor-associated epitope

<400>  1

Gln Pro Pro Asp Trp Leu Gln Gly His Tyr Leu Val Val Arg Tyr Glu
1               5                   10                  15

Asp Leu

<210>  2
<211>  19
<212>  PRT
<213>  Artificial sequence

<220>
<223>  Tumor-associated epitope

<400>  2

Tyr Pro Asp Arg Pro Gly Trp Leu Arg Tyr Ile Gln Arg Thr Pro Tyr
1               5                   10                  15

Ser Asp Gly

<210>  3
<211>  22
<212>  PRT
<213>  Artificial sequence

<220>
<223>  Tumor-associated epitope

<400>  3

Ala Lys Pro Glu Ala Ser Phe Gln Val Trp Asn Lys Asp Ser Ser Ser
1               5                   10                  15

Lys Asn Leu Ile Pro Arg
            20

<210>  4
<211>  18
<212>  PRT
<213>  Artificial sequence

<220>
<223>  Tumor-associated epitope

<400>  4

Asp His Ala Gln Leu Val Ala Ile Lys Thr Leu Lys Asp Tyr Asn Asn
1               5                   10                  15

Pro Gln

<210>  5
<211>  17
<212>  PRT
<213>  Artificial sequence

<220>
<223>  Tumor-associated epitope

<400>  5

Glu Pro Asn Lys Lys Phe Phe Glu Leu Val Gly Arg Thr Phe Asp Trp
1               5                   10                  15

His

<210>  6
<211>  16
<212>  PRT
<213>  Artificial sequence

<220>
<223>  Tumor-associated epitope

<400>  6

Leu Leu Leu Ile Leu Arg Asp Pro Ser Glu Arg Val Leu Ser Asp Tyr
1               5                   10                  15

<210>  7
<211>  9
<212>  PRT
<213>  Artificial sequence

<220>
<223>  Tumor-associated epitope

<400>  7

Gly Tyr Met Pro Tyr Leu Asn Arg Phe
1               5

<210>  8
<211>  10
<212>  PRT
<213>  Artificial sequence

<220>
<223>  Tumor-associated epitope

<400>  8

Lys Tyr Ser Lys Ala Leu Ile Asp Tyr Phe
1               5                   10

<210>  9
<211>  9
<212>  PRT
<213>  Artificial sequence

<220>
<223>  Tumor-associated epitope

<400>  9

Val Tyr His Ser Asp Ile Pro Lys Trp
1               5

<210>  10
<211>  9
<212>  PRT
<213>  Artificial sequence

<220>
<223>  Tumor-associated epitope

<400>  10

Phe Tyr Thr Leu Ile Pro His Asp Phe
1               5

<210>  11
<211>  10
<212>  PRT
<213>  Artificial sequence

<220>
<223>  Tumor-associated epitope

<400>  11

Ile Tyr Gly Lys Asp Val Phe Glu Ala Phe
1               5                   10

<210>  12
<211>  9

<212>  PRT
<213>  Artificial sequence

<220>
<223>  Tumor-associated epitope

<400>  12

Ile Phe Leu Thr Lys Ser Thr Lys Leu
1               5


<210>  13
<211>  9
<212>  PRT
<213>  Artificial sequence

<220>
<223>  Tumor-associated epitope

<400>  13

Arg His Thr Gly Ala Leu Pro Leu Phe
1               5


<210>  14
<211>  9
<212>  PRT
<213>  Artificial sequence

<220>
<223>  Tumor-associated epitope

<400>  14

Arg Pro Lys Glu Asn Val Thr Ile Met
1               5


<210>  15
<211>  9
<212>  PRT
<213>  Artificial sequence

<220>
<223>  Tumor-associated epitope

<400>  15

Leu Pro Arg Leu Glu Ala Leu Asp Leu
1               5


<210>  16
<211>  9
<212>  PRT
<213>  Artificial sequence

<220>
<223>  Tumor-associated epitope

<400>  16

Ser Pro Gly Gly Ala His Ser Asn Leu
1               5

<210>  17
<211>  9
<212>  PRT
<213>  Artificial sequence

<220>
<223>  Tumor-associated epitope

<400>  17

Val Pro Arg Asn Leu Pro Ser Ser Leu
1               5

<210>  18
<211>  9
<212>  PRT
<213>  Artificial sequence

<220>
<223>  Tumor-associated epitope

<400>  18

Leu Ile Trp Pro Leu Leu Ser Thr Val
1               5

<210>  19
<211>  9
<212>  PRT
<213>  Artificial sequence

<220>
<223>  Tumor-associated epitope

<400>  19

Val Ile Ala Glu Leu Pro Pro Lys Val
1               5

<210>  20
<211>  9
<212>  PRT
<213>  Artificial sequence

<220>
<223>  Tumor-associated epitope

<400>  20

Ala Leu His Arg Pro Asp Val Tyr Leu
1               5